(19) Europäisches Patentamt
European Patent Office
Office européen des brevets

(11) **EP 4 044 918 B1**

(12) **EUROPÄISCHE PATENTSCHRIFT**

(45) Veröffentlichungstag und Bekanntmachung des Hinweises auf die Patenterteilung:
**01.11.2023   Patentblatt 2023/44**

(21) Anmeldenummer: **20780904.7**

(22) Anmeldetag: **24.09.2020**

(51) Internationale Patentklassifikation (IPC):
**A61B 5/11** (2006.01)     **A61B 5/00** (2006.01)

(52) Gemeinsame Patentklassifikation (CPC):
**A61B 5/1116; A61B 5/1114; A61B 5/6892; A61B 5/6894;** A61B 2562/0247; A61B 2562/0252; A61B 2562/0261; A61B 2562/046; A61B 2562/168

(86) Internationale Anmeldenummer:
**PCT/AT2020/060343**

(87) Internationale Veröffentlichungsnummer:
**WO 2021/072461 (22.04.2021 Gazette 2021/16)**

(54) **VERFAHREN ZUR ORTS- UND LAGEBESTIMMUNG EINES BECKENS EINER PERSON**

METHOD FOR DETERMINING THE LOCATION AND POSITION OF A PERSON'S PELVIS

PROCÉDÉ PERMETTANT DE DÉTERMINER L'EMPLACEMENT ET LA POSITION DU BASSIN D'UNE PERSONNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.10.2019   AT 508952019**

(43) Veröffentlichungstag der Anmeldung:
**24.08.2022   Patentblatt 2022/34**

(73) Patentinhaber: **Sanlas Holding GmbH**
**8010 Graz (AT)**

(72) Erfinder: **ZENZMAIER, Cornelia**
**8301 Laßnitzhöhe (AT)**

(74) Vertreter: **Röggla, Harald**
**Schwarz & Partner**
**Patentanwälte**
**Wipplingerstraße 30**
**1010 Wien (AT)**

(56) Entgegenhaltungen:
JP-A- 2002 005 761

• VAN GEFFEN P ET AL: "Decoupled pelvis rotation in sitting: A passive motion technique that regulates buttock load associated with pressure ulcer development", JOURNAL OF BIOMECHANICS, PERGAMON PRESS, NEW YORK, NY, US, Bd. 42, Nr. 9, 19. Juni 2009 (2009-06-19), Seiten 1288-1294, XP026172091, ISSN: 0021-9290, DOI: 10.1016/J.JBIOMECH.2009.03.010 [gefunden am 2009-04-22]

EP 4 044 918 B1

**Beschreibung**

Die vorliegende Erfindung betrifft ein Verfahren und eine Vorrichtung zur Orts- und Lagebestimmung eines Beckens einer auf einem Sitz sitzenden oder auf einer Liege oder einer Auflage sitzenden oder liegenden Person.

Das Becken einer Person gilt als Stellungsregler für eine neutrale Ausrichtung der funktionellen Wirbelsäule und der unteren Extremitäten. Obwohl das Becken alters- und geschlechtsspezifische Unterschiede hinsichtlich der Längen- und Winkelverhältnisse aufweist, kann jedoch eine allgemein gültige einheitliche Beschreibung für dessen lagebedingte Belastung erfolgen. Bei Sitzsystemen wird das Becken der Person im Allgemeinen von der Sitzfläche und der Rückenlehne des Sitzsystems gestützt. Die Sitzbeinhöcker dienen hierbei zur Aufnahme des Oberkörpergewichts und Anteilen der unteren Extremitäten und können bei einer ergonomisch richtigen im Wesentlichen aufrechten Sitzposition der Person hohen Sitzdruck aufnehmen, wohingegen das Steißbein und das Kreuzbein bei dieser aufrechten Sitzposition im Wesentlichen unbelastet bleiben. Bei Liegesystemen in einer ergonomisch richtigen horizontalen Rückenlage der Person stützen die Wirbelsäule und das Kreuzbein die Oberkörpergewichtskraft ab, wobei der von der Gewichtskraft der Person ausgeübte Auflagedruck im Beckenbereich am Kreuzbein im Wesentlichen maximal ist und das Steißbein im Wesentlichen unbelastet bleibt. Bei einer ergonomisch richtigen Bauchlage übernimmt das Schambein der Person den maximalen Anteil jenes Körperabschnittsgewichts im Beckenbereich.

Personen sind sich ihrer eigenen individuellen Körperhaltung im Stehen, Sitzen und Liegen jedoch oft nicht bewusst und nehmen Positionen ein, die einseitige schmerzhafte Muskelkontrakturen verursachen und langfristig bei Fehlbelastung der Wirbelsäulen- und Beckenstruktur, insbesondere in dessen Übergangsbereichen, den Iliosakralgelenken, zu Abnutzungsprozessen, auch Bandscheibenvorfällen, oder zumindest zu Verpressungen und Stauchungen, insbesondere der Wirbelsäule, führen können. Besonders langes Sitzen erfordert eine permanente Haltearbeit der Muskulatur oft in Fehlhaltung und ohne ausreichende Ausgleichsbewegungen, wodurch muskuläre Dysbalancen hervorgerufen und vielfältige körperliche Beschwerden, auch in den Extremitäten, begünstigt werden.

Dem Stand der Technik sind zahlreiche Mittel und Methoden bekannt, die versuchen, eine ergonomisch richtige Körperhaltung herzustellen und fortwährend zu bewahren. So zeigen DE 10 2012 017 681 A1, US 2002/0193707 A1 und EP 1 093 755 A1 beispielsweise Verfahren und Vorrichtungen zur Messung von Auflagedrücken einer Person beim Sitzen bzw. beim Liegen. Mit solchen Verfahren bzw. Vorrichtungen ist es jedoch nicht möglich eine Lage- und Ortsbestimmung des Beckens einer Person durchzuführen.

Die Patentanmeldung A 50386/2019, welche zum Zeitpunkt der vorliegenden Patentanmeldung noch nicht veröffentlicht ist, offenbart zum Beispiel eine Vorrichtung zur Körperpositionierung einer Person mit Stellelementen und Sensoren, die auf einem Sitzelement und auf einem Rückenelement angeordnet sind. In dieser Patentanmeldung ist der Abrollvorgang der Person während deren Überführung von einer aufrechten in eine horizontale Liegeposition erwähnt, wobei das Becken der Person durch lineare Stellbewegungen durch Rotationen um die Sagittal-, Horizontal- und Longitudinalachse positioniert wird.

In der Patentanmeldung A 50386/2019 wird die Lage des Beckens nur indirekt und ungenügend genau bestimmt, da das Becken der Person bereits positioniert wird, sobald ein vorgegebener Sitz- und/oder Auflagedruckunterschied erreicht wird. Zudem erfasst diese Vorrichtung keine gemischten Bewegungen, d. h. Bewegungen, die um zumindest zwei Körperachsen der Person gleichzeitig erfolgen, sondern lediglich eine Bewegung ausschließlich um die Sagittal-, Horizontal- oder Longitudinalachse. Um eine ergonomische Positionierung der Person auf dem Sitz- oder Liegesystem zu erzielen, ist es jedoch notwendig die Lage des Beckens in jeder Position der Person auf dem Sitz- oder Liegesystem exakt zu bestimmen.

Die Offenbarung Van Geffen et al., J. Biomech. 42 (2009) 1288-1295 zeigt eine Vorrichtung und ein Verfahren zur Orts- und Lagebestimmung eines Beckens einer auf einem Sitz sitzenden Person. Bei dem Verfahren werden die von den Sitzbeinhöckern und dem Steißbein der Person ausgeübten Sitzdrücke durch einen am Sitz angeordneten Flächensensor erfasst. Die Schrift JP 2002 005761 A zeigt ein ähnliches Verfahren, bei dem die Positionen der Sitzbeinhöcker aus den Spitzendrücken der Druckverteilung mittels einer Rechnereinheit bestimmt wird und so der Abstand zwischen den Sitzbeinhöckern berechnet wird.

Die Aufgabe der vorliegenden Erfindung ist es, ein Verfahren und eine Vorrichtung zur Lage- und Ortsbestimmung des Beckens einer Person bereitzustellen, welches in ein bereits bestehendes Sitzsystem oder Liegesystem oder eine Auflage integrierbar ist und welches die Nachteile des Standes der Technik vermeidet.

Die vorliegende Erfindung löst die gestellten Aufgaben durch Bereitstellen eines Verfahrens zur Lage- und Ortsbestimmung eines Beckens einer Person gemäß Anspruch 1 und einer Vorrichtung zur Lage- und Ortsbestimmung eines Beckens einer Person mit den Merkmalen des Anspruchs 5.

In einem weiteren Aspekt stellt die Erfindung ein Computerprogrammprodukt gemäß Anspruch 8 bereit, und einen computerlesbaren Datenträger gemäß Anspruch 9, auf dem das

Computerprogrammprodukt gespeichert ist.

Das erfindungsgemäße Verfahren umfasst die folgenden Schritte: das Erfassen von von den Sitzbeinhöckern und dem Steißbein der im Wesentlichen aufrecht sitzenden Person oder den Beckenkämmen und dem Schambein der in Bauchlage liegenden Person auf den Sitz, die Liege oder die Auflage ausgeübten Sitzdrücke/Auflagedrücke durch einen an dem Sitz, der Liege oder der Auflage angeordneten Flächensensor; das Ermitteln einer ersten Position auf dem Flächensensor durch eine Rechnereinheit, bei der ein von einem ersten

Sitzbeinhöcker/Beckenkamm der Person ausgeübter erster Spitzensitzdruck/Spitzenauflagedruck erreicht wird; das Ermitteln einer zweiten Position auf dem Flächensensor durch die Rechnereinheit, bei der ein von einem zweiten Sitzbeinhöcker/Beckenkamm der Person ausgeübter zweiter

Spitzensitzdruck/Spitzenauflagedruck erreicht wird, wobei die zweite Position in einem vorbestimmten Abstandbereich zu der ersten Position liegt; das Ermitteln eines Abstandes zwischen der ersten Position und der zweiten Position durch die Rechnereinheit; das Ermitteln einer dritten Position auf dem Flächensensor durch die Rechnereinheit, bei der ein von dem Steißbein/Schambein der Person ausgeübter dritter Spitzensitzdruck/Spitzenauflageruck erreicht wird, wobei die dritte Position zwischen der ersten Position und der zweiten Position liegt, und wobei die dritte Position im Wesentlichen auf deren gemeinsamen Verbindungslinie liegt oder in einem vorbestimmten Abstandsbereich auf einer im Wesentlichen senkrecht zu der Verbindungslinie verlaufenden Linie liegt; das Eingrenzen eines Sitz- und Auflagedruck detektierenden Bereichs des Flächensensors durch die Rechnereinheit; und das Auswerten der Sitz- und Auflagedrücke des Beckens der Person in dem eingeschränkten Sitz- und Auflagedruck detektierenden Bereich durch die Rechnereinheit, wobei die Rechnereinheit zu dem Einschränken des Sitz- und Auflagedruck detektierenden Bereichs einen iterativen Algorithmus ausführt, der die Mandelbrot-Menge abbildet.

Durch das erfindungsgemäße Verfahren ist es möglich, zu jeder Zeit die Lage und die Position des Beckens der Person auf einem Sitz oder einer Liege zu bestimmen, wodurch nicht nur einzelne Bewegungen der Person um eine ihrer Körperachsen erfasst werden können, sondern insbesondere auch gemischte Bewegungen der eingangs erwähnten Art. Hierfür werden die oben genannten drei Positionen und die Größe der von dem Becken der Person auf den Flächensensor ausgeübten Sitz- und Auflagedrücke bestimmt. Dies wird bevorzugt durch eine Mustererkennung der Maxima der Sitz- und Auflagedrücke durch die Rechnereinheit durchgeführt. Vorteilhaft ist, dass durch die Maxima der Sitz- und Auflagedrücke zudem Rückschlüsse auf die Gewichtskraft des Oberkörpers, und je nach Ausführung des Sitzes mit oder ohne Armlehnen inklusive/exklusive Gewichtskraft der oberen Extremitäten der Person gemacht werden können, da in einer im Wesentlichen aufrechten Sitzposition die Sitzbeinhöcker einen Großteil des Oberkörpergewichts auf den Flächensensor übertragen. Kleinere Gewichtskraftanteile, welche in dieser Sitzposition von den unteren Extremitäten der Person auf den Flächensensor übertragen werden und sich in Form eines Offsets bei den Sitz- und Auflagedrücken bemerkbar machen, können durch die Rechnereinheit aufgrund des eingeschränkten detektierenden Bereichs kompensiert werden. Bei einer Anordnung des Flächensensors an einem Rückenelement eines Sitzes, insbesondere für rückgeneigte Positionen des Rückenelements, können zusätzlich Druckschwankungen, die durch die Respiration der Person verursacht werden, dynamisch von dem Flächensensor erfasst werden. Hierdurch können Veränderungen in der Atmung, beispielsweise eine Tachypnoe, erfasst werden.

Da die Sitz- und Auflagedrücke des Beckens, des Steiß- und Kreuzbeines ausschließlich in dem Sitz- und Auflagedruck detektierenden Bereich erfasst werden, ist der Vorteil enthalten, dass auf andere Bereiche des Flächensensors ausgeübte Drücke, die von dem Becken der Person auf dem Flächensensor ausgeübten Sitz- und Auflagedrücke nicht beeinflussen. Wird die Vorrichtung beispielsweise in Krankenbetten eingesetzt, haben auf dem Krankenbett befindliche Gegenstände, z. B. Taschen von Besuchern, Equipment von Krankenpflegern oder Ärzten, keinen Einfluss auf die Auswertung der Sitz- und Auflagedrücke des Beckens des auf dem Krankenbett befindlichen Patienten, da diese im Wesentlichen die Geometrie des Beckens berücksichtigt und auch von zum Teil fixierten Fehlhaltungen der Wirbelsäule, die eine Abstützung an der Rückenlehne bedingen, unbeeinflusst bleibt. Für jede Position der Person auf dem Flächensensor kann somit die Lage und die Position des Beckens präzise bestimmt werden.

Ein weiterer Vorteil ist, dass durch das erfindungsgemäße Verfahren unabhängig eines lagebedingten Gewichtskrafteinflusses und unabhängig einer Abstützung des Oberkörpergewichts der Person am Rückenelement, ein Relativwinkel zwischen dem Sitzelement, insbesondere dessen Sitzfläche, und dem Rückenelement bestimmt werden kann. Dies wird dadurch erreicht, dass jedem in dem Sitz- und Auflagedruck detektierenden Bereich des Flächensensors auftretenden Sitz- und Auflagedruck eine Position durch die Rechnereinheit zuweisbar ist, und somit eine Druckverteilung für jede Position des Sitz- und Auflagedruckes bekannt ist.

Um den Sitz- und Auflagedruck detektierenden Bereich des Flächensensors eingrenzen zu können, führt die Rechnereinheit einen iterativen Algorithmus aus, der die Mandelbrot-Menge mittels einer rekursiven Folge $z_{n+1} = z_n^2 + C$, mit einer Anfangsbedingung $z_0 = 0$, abbildet. Hierfür wird der Flächensensor als Gauß'sche Ebene aufgefasst, bei welcher die von dem Becken der Person ausgeübten Sitz- und Auflagedrücke Punkte C in der Gauß'schen Ebene sind. Durch das Einsetzen von unterschiedlichen Punkten C in die rekursive Folge, kann herausgefunden werden, welche Punkte C der Gauß'schen Ebene zur Mandelbrot-Menge gehören und welche nicht. Zur Mandelbrot Menge gehören hierbei jene Punkte, für die die Folge beschränkt bleibt, also konvergiert, d. h. sich immer mehr einem Grenzwert annähert. Für einen Punkt C in der Gauß'schen Zahlenebene mit den Koordinaten C(0/0) ergibt sich ein kreisförmiger Sitz- und Auflagedruck detektierender Bereich, da die Folge sich zu $z_{n+1} = z_n^2$ reduziert. Bei Punkten C außerhalb dieses kreisförmigen Bereichs streben die Zahlen gegen unendlich und die Folge ist nicht mehr beschränkt. Durch das Abbilden der Mandelbrot-Menge durch die rekursive Folge ist es möglich, die dreidimensionale Struktur des Beckens der Person zweidimensional auf einer ebenen Kontaktfläche, beispielsweise auf dem Flächensensor, abzubilden. Die zweidimensionale Struktur des Beckens entspricht dabei im Wesentlichen der Form einer Kardioide, welche durch die Mandelbrot-Menge darstellbar ist. Die zweidimensionale kardioide Form des Beckens entsteht durch den Abrollvorgang dessen auf der ebenen Kontaktfläche. Ausgehend von einer ersten Lage des Beckens, welche einer aufrechten Sitzposition der Person entspricht, wird das Becken dieser Person 90° um die Horizontalachse in eine zweite Lage nach vorne und anschließend in eine dritte Lage nach hinten gekippt. Zum Erreichen der dritten Lage wird das Becken 90° bezüglich der ersten Lage des Beckens beziehungsweise 180° bezüglich der zweiten Lage des Beckens um die Horizontalachse nach hinten gekippt. Die dritte Lage entspricht einer horizontalen Rückenlage der Person. Von der horizontalen Rückenlage ausgehend wird das Becken zu beiden Seiten über die Beckenkämme abgerollt, was einer gemischten Rotation des Beckens oder einer reinen Rotation um die Longitudinalachse in Liegeposition entspricht. So entsteht bei der Verbindung aller Kontaktpunkte an der ebenen Kontaktfläche die Form einer Kardioide. Die Kardioide entspricht somit einer zweidimensionalen Darstellung einer Außenkontur des dreidimensionalen Beckens der Person. Die sakrale Struktur des Beckens wird hierbei ebenfalls zweidimensional in dem Inneren der Kardioide abgebildet.

Abhängig von der Anzahl der Iterationen, welche die Form und Größe des Sitz- und Auflagedruck detektierenden Bereichs und somit die Form und Größe der Mandelbrot-Menge bestimmt, treten die von dem Becken der Person auf dem Flächensensor ausgeübten Sitz- und Auflagedrücke innerhalb dieser Kardioide auf. Wenige Iterationen, beispielsweise zwei, führen zu einem größeren Sitz- und Auflagedruck detektierenden Bereich, in diesem Fall zu einem ellipsenförmigen Bereich, wohingegen eine große Anzahl von Iterationen einen eingeschränkteren, beispielsweise einen kardioideförmigen, Sitz- und Auflagedruck detektierenden Bereich erzeugt. Für die Positionierung des Beckens der Person durch den Stand der Technik bekannte Mittel und Methoden, beispielsweis jenen von A 50386/2019, ist eine höhere Anzahl von Iterationen einer geringeren Anzahl vorzuziehen. Bei bereits fünf Iterationen ist ein kardioideförmiger eingeschränkter Sitz- und Auflagedruck detektierender Bereich abbildbar, welcher für die Positionierung des Beckens durch die oben erwähnten Mittel und Methoden genügt. Bei dem Erreichen einer vorgegebenen Anzahl wird die momentan durchlaufende Iteration unterbrochen. Die genannte Anzahl von Iterationen kann beispielsweise von Dritten, z. B. von Fachpersonal wie Krankenpflegern oder Ärzten, vorgegeben und an der Rechnereinheit eingestellt werden. Die Anzahl der Iterationen ist jedoch vorwiegend von dem Einsatzzweck der Vorrichtung abhängig. In Operationsbetten integriert, ist es notwendig die Sitz- und Auflagedrücke möglichst exakt zu erfassen, weshalb hierfür eine höhere Anzahl von Iterationen gewählt wird.

Die oben genannten erfindungsgemäßen Schritte werden durch ein Computerprogrammprodukt realisiert, welches Befehle umfasst, die bei der Ausführung des Programms durch die Rechnereinheit diese veranlassen, diese Schritte auszuführen. Das Computerprogrammprodukt ist hierbei auf einem computerlesbaren Datenträger gespeichert. Mit Unterstützung des Programms können Einstellungen an der Vorrichtung vorgenommen werden, die es erlauben, die Vorrichtung für deren jeweiligen Einsatzzweck anzupassen, wie zum Beispiel die zuvor erwähnte Einstellung über die Anzahl der Iterationen des Algorithmus.

Die Vorrichtung zur Orts- und Lagebestimmung eines Beckens einer auf einem Sitz sitzenden oder auf einer Liege oder einer Auflage sitzenden oder liegenden Person umfasst eine Rechnereinheit und einen mit der Rechnereinheit verbundenen Flächensensor. Der Flächensensor ist auf dem Sitz, der Liege oder der Auflage angeordnet und dazu ausgebildet, Sitz- und Auflagedrücke, die von dem Becken, dem Steiß- und Kreuzbein der Person auf den Flächensensor ausgeübt werden, zu erfassen, wobei die Rechnereinheit dazu ausgebildet ist, die Schritte des oben genannten erfindungsgemäßen Verfahrens auszuführen.

Vorteilhaft ist, dass die Vorrichtung in bestehende Sitze oder Liegen integrierbar oder als Sitzauflage auf einen Stuhl oder Bett verwendbar ist. Hierbei ergeben sich eine Vielzahl von Anwendungsmöglichkeiten, wobei die Vorrichtung nicht auf folgende Beispiele beschränkt ist. Die Vorrichtung könnte Beispiele angebend in folgende Einrichtungen integriert werden: in Bürosessel, Rollstühle, Fahrzeugsitze, Kinderrückhaltesysteme, Trainingsgeräte, Behandlungsliegen, Matratzen und/oder im Rehabilitations- und Therapiebereich verwendet werden, insbesondere in Stehbetten, Stehbrettern und/oder Operationsbetten. Die Integration der gegenständlichen Erfindung in Operationsbetten ist vor allem für in Bauchlage oder in Rückenlage durchgeführte Eingriffe sinnvoll, da diese oft mit geneigten Operationsbetten durchgeführt werden und es hierdurch zu einer unerwünschten Verlagerung der auf dem Operationsbett befindlichen Person kommen kann beziehungsweise zu Folgen einer Fehlpositionierung des Beckens in Bezug zur neutralen Ausrichtung der Wirbelsäule, bei der die Wirbelsäule, insbesondere im Lumbalbereich in eine gestauchte oder gestreckte Lage gezwungen wird. Ein Eingriff bei einem derart positionierten Becken kann zu einer Vielzahl von Problemen führen. Durch die gegenständliche Erfindung kann eine Verschiebung oder Rotation des Beckens der Person rasch durch Erfassen von Verschiebungen insbesondere der maximalen Sitz- und Auflagedrücke auf dem Flächensensor erkannt werden. Durch den Stand der Technik bekannte Mittel und Methoden, beispielsweise durch die eingangs zitierte Vorrichtung, können Verschiebungen beziehungsweise Rotationen des Beckens entgegengewirkt und die ursprüngliche Position des Beckens neuerlich hergestellt werden. Im Zuge einer Operation kann ein Eingriff somit mit bestmöglicher Präzision durchgeführt und mögliche Operationsrisiken beziehungsweise schmerzhafte Folgen einer Fehlpositionierung der funktionellen Wirbelsäule, insbesondere durch eine auf die Beckenstellung abgestimmte Stützung der Lendenwirbelsäule minimiert oder sogar ausgeschlossen werden.

In einer bevorzugten Ausführungsform der Erfindung ist der Flächensensor ein Array von Sensoren, die ausgewählt sind aus der Gruppe bestehend aus mechanischen, elektrischen, pneumatischen oder hydraulischen Sensoren. Hierdurch ist die Position des Beckens in jeder Position der Person auf dem Sitz oder der Liege detektierbar. Durch ein Variieren der Anzahl der Sensoren kann die Genauigkeit des Erfassens der Sitz- und Auflagedrücke durch die Sensoren beeinflusst werden. Eine große Anzahl von Sensoren ermöglicht ein präzises Erfassen der Sitz- und Auflagedrücke des Beckens der Person.

In einer bevorzugten Ausführungsform der Erfindung ist der Flächensensor so geformt ist, dass dieser sich wenigstens teilweise dem Becken der Person anpasst. Sitz- und Auflagedrücke können hierdurch mit einer höheren Empfindlichkeit erfasst werden, wodurch bereits leichte Schwerpunktverschiebungen, insbesondere im Bereich des pelvinen Körperabschnitts, von den Sensoren detektiert werden können.

Die Erfindung wird nun unter Bezugnahme auf die Zeichnungen anhand von nicht einschränkenden Ausführungsbeispielen näher erläutert.

Fig. 1 zeigt ein Blockdiagramm, welches Schritte eines Verfahrens gemäß Anspruch 1 der Erfindung in einer bevorzugten Reihenfolge darstellt.
Fig. 2 zeigt eine schematische perspektivische Darstellung eines Sitzes mit einem integrierten Flächensensor gemäß Anspruch 8 der Erfindung.
Fig. 3 zeigt eine schematische Darstellung einer Sitzfläche des in Fig. 1 veranschaulichten Sitzes, in welchem eine erste Position, eine zweite Position und eine dritte Position gemäß Anspruch 1 der Erfindung dargestellt sind.
Fig. 4 zeigt einen Sitz- und Auflagedruck detektierenden Bereich des Flächensensors.

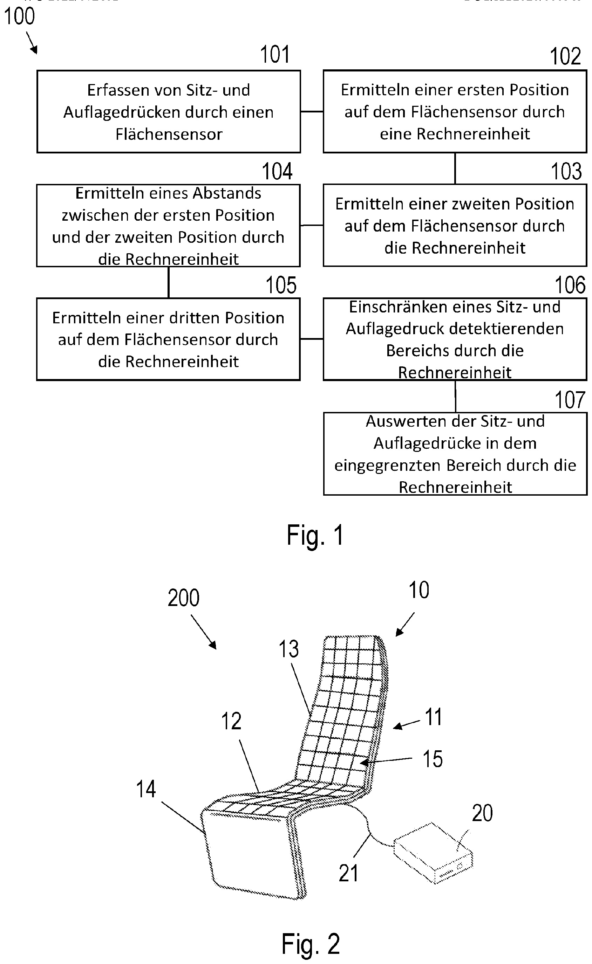

Nachfolgend wird auf die Fig. 1 bis 3 Bezug genommen. Zunächst bezugnehmend auf Fig. 2 zeigt diese eine erfindungsgemäße Vorrichtung 200 zur Orts- und Lagebestimmung eines Beckens einer auf einem Sitz 10 sitzenden oder auf einer Liege oder einer Auflage sitzenden oder liegenden Person. Die Liege, die Auflage und die Person sind in den Figuren nicht dargestellt. Die Vorrichtung 200 umfasst eine Rechnereinheit 20 und einen mit der Rechnereinheit 20 verbundenen Flächensensor 11. Der Flächensensor 11 ist auf einem Sitzelement 12 und in einem Rückenelement 13 des Sitzes 10 angeordnet, insbesondere in diesem integriert, wobei ein Beinelement 14 des Sitzes 10 keinen Flächensensor 11 aufweist. In einer weiteren Ausführungsform, welche nicht dargestellt ist, kann zusätzlich zu dem Sitzelement 12 und dem Rückenelement 13, auch das Beinelement 14 einen Flächensensor 11 aufweisen. In noch weiteren Ausführungsformen, welche ebenfalls nicht dargestellt sind, kann ausschließlich das Sitzelement 12 oder das Rückenelement 13 einen Flächensensor 11 aufweisen. Die genannten Ausführungsformen sind kombinierbar, d. h., dass beispielsweise das Beinelement 14 und das Sitzelement 12 einen Flächensensor 11 aufweisen und das Rückenelement 13 keinen Flächensensor 11 aufweist. Der in Fig. 2 beispielhaft dargestellte Flächensensor 11 weist relativ großflächige Einzelsensoren auf, weshalb die Bestimmung von Abständen nur relativ grob erfolgen kann. Die im Zusammenhang mit der Erfindung verwendeten Flächensensoren können daher auch viel mehr Einzelsensoren in einem feineren Raster bzw. Array aufweisen, als dies in Fig. 2 dargestellt ist.

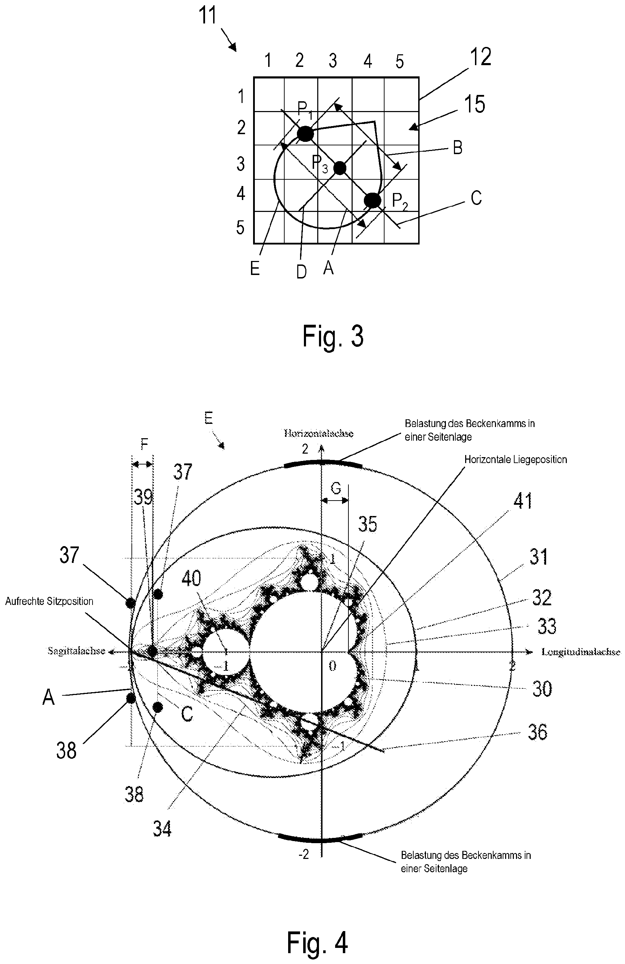

Um eine hohe Empfindlichkeit der Sensoren 15 zu erreichen, ist der Flächensensor 11 so geformt, dass dieser sich wenigstens teilweise dem Becken der Person anpasst. Hierdurch kann der Flächensensor 11, der dazu ausgebildet ist von dem Becken, des Steiß- und Kreuzbeines der Person auf den Flächensensor 11 ausgeübte Sitz- und Auflagedrücke zu erfassen, die Sitz- und Auflagedrücke mit einer hohen Präzision erfassen. Wie in Fig. 2 ersichtlich, ist der Flächensensor 11 ein Array von Sensoren 15, die ausgewählt sind aus der Gruppe bestehend aus mechanischen, elektrischen, pneumatischen oder hydraulischen Sensoren. Insbesondere ist der Flächensensor ein zweidimensionales Array von Sensoren 15, welches das Sitzelement 12 und das Rückenelement 14 des Sitzes 10 vollständig einnimmt. Die Anzahl der im Array angeordneten Sensoren 15 kann variieren und ist nicht auf eine bestimmte Zahl beschränkt. In Fig. 3 ist ein Array mit 5x5 Sensoren 15 dargestellt. Hierdurch kann die Position der von dem Becken, des Steiß- und Kreuzbeines der Person ausgeübten Sitz- und Auflagedrücke und somit jede Position des Beckens der Person ausreichend genau bestimmt werden. Die Sensoren 15 des Flächensensors 11 sind bevorzugt als flache Kammern ausgebildet, welche mit einem Fluid, beispielsweise Luft oder Wasser, gefüllt sind. Der Flächensensor 11 ist hierbei mit der Rechnereinheit 20 über in den Figuren nicht dargestellte fluidführende Kanäle verbunden. Zur Erfassung der Sitz- und Auflagedrücke können auch elektrische oder mechanische Sensoren eingesetzt werden, und sind deshalb nicht auf die zuvor beispielhaft genannten Sensoren beschränkt. So können die Sensoren 15 auch in einem Array anordenbare Dehnungsmessstreifen sein. Die Rechnereinheit 20 ist hierbei elektrisch mit dem Flächensensor 11 verbunden.

Im Folgenden wird auf Figur 1 und 3 Bezug genommen. In Fig. 1 ist ein Blockdiagramm dargestellt, welches Schritte 101, 102, 103, 104, 105, 106, 107 eines erfindungsgemäßen Verfahrens 100 zur Orts- und Lagebestimmung eines Beckens einer auf einem Sitz 10 sitzenden oder auf einer Liege oder einer Auflage sitzenden oder liegenden Person darstellt. In einem ersten Schritt 101 des Verfahren 100 werden von den Sitzbeinhöckern und dem Steißbein der im Wesentlichen aufrecht sitzenden Person oder den Beckenkämmen und dem Schambein der in Bauchlage liegenden Person auf den Sitz 10, die Liege oder die Auflage ausgeübten Sitzdrücke/Auflagedrücke durch einen an dem Sitz 10, der Liege oder der Auflage angeordneten Flächensensor 11 erfasst. In einem nächsten Schritt 102 wird durch die Rechnereinheit 20 eine erste Position $P_1$ auf dem Flächensensor 11 ermittelt, bei der ein von einem ersten Sitzbeinhöcker/Beckenkamm der Person ausgeübter erster Spitzensitzdruck/Spitzenauflagedruck erreicht wird. Die erste Position $P_1$ wird beispielhaft in Fig. 3 von dem Sensor in der Zeile 2 und Spalte 2 des Arrays erfasst. In einem weiteren Schritt 103 wird eine zweite Position $P_2$ auf dem Flächensensor 11 durch die Rechnereinheit 20, bei der ein von einem zweiten Sitzbeinhöcker/Beckenkamm der Person ausgeübter zweiter Spitzensitzdruck/Spitzenauflagedruck erreicht wird, wobei die zweite Position $P_2$ in einem vorbestimmten Abstandbereich A zu der ersten Position $P_1$ liegt. Die zweite Position $P_2$ wird in Fig. 3 von dem Sensor in der Zeile 4 und Spalte 4 des Arrays erfasst. Der vorbestimmte Abstandbereich A ist durch die geometrische Anordnung der Sitzbeinhöcker beziehungsweise der Beckenkämme der Person gegeben und beträgt für die Sitzbeinhöcker 100 mm bis 150 mm und für die Beckenkämme in Bauchlage 200 mm bis 250 mm. Der angeführte Abstandbereich A gilt als beispielhaft, da neben geschlechtsspezifischen Unterschieden auch weitere Größen wie das Alter oder pathologische Veränderungen, insbesondere im Bereich des Steißbeines einer Person, einen Einfluss auf die Geometrie des Beckens beziehungsweise die sakrale Struktur, und somit auf den Abstandbereich A haben. In einem darauffolgenden Schritt 104 des Verfahrens 100 wird durch die Rechnereinheit 20 ein Abstand B zwischen der ersten Position $P_1$ und der zweiten Position $P_2$ ermittelt. Dieser Abstand B entspricht dabei einem wahren Abstand zwischen dem ersten Sitzbeinhöcker/Beckenkamm und dem zweiten Sitzbeinhöcker/Beckenkamm der Person, wobei der Abstand B zwischen den Beckenkämmen größer ist als zwischen den Sitzbeinhöckern. Unter dem Begriff "wahren Abstand" ist der dem jeweiligen Becken der Person entsprechende tatsächliche Abstand zwischen den Sitzbeinhöckern beziehungsweise den Beckenkämmen zu verstehen, der von dem Flächensensor 11 erfasst wird.

Wurde die erste Position $P_1$, die zweite Position $P_2$ und der Abstand B zwischen diesen Positionen $P_1$, $P_2$ durch die Rechnereinheit 20 bestimmt, wird durch die Rechnereinheit 20 anschließend eine dritte Position $P_3$ auf dem Flächensensor 11 ermittelt, bei der ein von dem Steißbein/Schambein der Person ausgeübter dritter Spitzensitzdruck/Spitzenauflagedruck erreicht wird. Die dritte Position $P_3$ liegt hierbei zwischen der ersten Position $P_1$ und der zweiten Position $P_2$, wobei die dritte Position $P_3$ im Wesentlichen auf deren gemeinsamen Verbindungslinie C liegt oder in einem vorbestimmten Abstandsbereich, nicht dargestellt, auf einer im Wesentlichen senkrecht zu der Verbindungslinie C verlaufenden Linie D liegt. Die dritte Position $P_3$ wird in Fig. 3 durch den Sensor in der Zeile 3 und Spalte 3 des Arrays erfasst.

Nach dem Ermitteln der dritten Position $P_3$ wird durch die Rechnereinheit 20 in einem nächsten Schritt 106 ein eingeschränkter Sitz- und Auflagedruck detektierender Bereich E ermittelt, wobei dieser in der Fig. 3 schematisch, und in Fig. 4 in Form einer Mandelbrot-Menge 30, dargestellt ist. Um den Sitz- und Auflagedruck detektierenden Bereich E einzuschränken, führt die Rechnereinheit 20 einen iterativen Algorithmus aus, der die genannte Mandelbrot-Menge 30 abbildet.

In einem letzten Schritt 107 des Verfahrens 100 werden die Sitz- und Auflagedrücke des Beckens der Person in dem eingeschränkten Sitz- und Auflagedruck detektierenden Bereich E durch die Rechnereinheit 20 ausgewertet. Sitz und Auflagedrücke außerhalb dieses Bereiches E werden von der Rechnereinheit 20 nicht weiter ausgewertet.

Die Rechnereinheit 20 der Vorrichtung 100 ist hierbei dazu ausgebildet, die Schritte 101, 102, 103, 104, 105, 106, 107 des oben angeführten Verfahrens 200 auszuführen, wobei die Reihenfolge des Verfahrens 100 nicht auf die in Fig. 1 dargestellte, bevorzugte Reihenfolge beschränkt ist. So können beispielsweise die Schritte 102, 103 des Verfahrens 100 auch in umgekehrter Reihenfolge stattfinden.

Im Folgenden wird auf Fig. 4 Bezug genommen. Fig. 4 zeigt den eingeschränkten Sitz- und Auflagedruck detektierenden Bereich E beziehungsweise die Mandelbrot-Menge 30 in unterschiedlicher Form und Größe. Fig. 4 zeigt des Weiteren die in dem eingeschränkten Sitz- und Auflagedruck detektierenden Bereich E auftretenden und vom Becken und dem Steiß- oder Kreuzbein auf den Flächensensor 11 ausgeübten Sitz- und Auflagedrücke 37, 38, 39 anhand derer der Ort und im Weitern auch die Lage des Beckens bestimmbar ist. Wie bereits erwähnt, wird der Sitz- und Auflagedruck detektierende Bereich E durch den zuvor genannten iterativen Algorithmus eingeschränkt. Aus Gründen der Veranschaulichung seien eine erste Iteration 31 des Algorithmus, die einen kreisförmigen Sitz- und Auflagedruck detektierenden Bereich E bildet, eine zweite Iteration 32, die einen ellipsenförmigen Sitz- und Auflagedruck detektierenden Bereich E bildet, und eine dritte Iteration 33 genannt. Wie in Fig. 4 ersichtlich, wird mit zunehmenden Iterationen der Sitz- und Auflagedruck detektierende Bereich E zunehmend eingeschränkt, wobei bereits bei fünf Iterationen 34 der Sitz- und Auflagedruck detektierende Bereich E im Wesentlichen die Form einer Kardioide beziehungsweise die Form der Mandelbrot-Menge 30 aufweist. Auf der Abszisse ist, dem rechtwinkeligen Koordinatensystem der Beckenstruktur entsprechend, die Sagittalachse, im Bereich -2 bis 0 und die Longitudinalachse im Bereich 0 bis 2, und auf der Ordinate die Horizontalachse des Beckens der Person aufgetragen. Ein Ursprung 35, dargestellt in einem Kreuzungspunkt der Abszisse und der Ordinate, stellt eine stabile Gleichgewichtslage mit Belastung der sakralen Struktur dar und ist in einem Abstand G von einem Rand 41 der Mandelbrot-Menge 30 entfernt dargestellt ist. Der Ursprung 35 entspricht dem Körperabschnittsgewichtschwerpunkt der Person und repräsentiert bei der Integration des Flächensensors 11 eine horizontale Rückenlage der Person beziehungsweise wird jener Punkt mit maximalem Auflagedruck durch das Kreuzbein beaufschlagt, sofern die Longitudinalachse beziehungsweise die Frontalebene des Oberkörpers der Person eine parallele Ausrichtung zu einer Stützstruktur, wie beispielsweise einem in den Figuren nicht dargestellten Stehbrett, aufweist.

Während eines Abrollvorgangs des Beckens, d. h. eine Rotation des Beckens um die Horizontalachse, üben, ausgehend von einer aufrechten Sitzposition der Person auf dem Flächensensor 11, zunächst die Sitzbeinhöcker Spitzensitzdrücke 37, 38 auf den Flächensensor 11 aus. Die Spitzensitzdrücke 37, 38 liegen hierbei in dem zuvor genannten Abstand A zueinander und treten außerhalb des eingeschränkten Sitz- und Auflagedruck detektierenden Bereiches E auf. Bei asymmetrischer Belastung der Sitzbeinhöcker können Druckunterschiede zwischen den Spitzensitzdrücke 37, 38 von dem Flächensensor 11 detektiert werden. Durch diese Druckunterschiede kann durch die Rechnereinheit 20 ein Rotationswinkel des Beckens um die Sagittalachse der Person bestimmt werden, wodurch die wahre Länge der Sitzbeinhöcker, im Fall einer Bauchlage die wahre Länge der Beckenkämme, und daraus ein Radius des kreisförmigen Sitz- und Auflagedruck detektierenden Bereichs E der ersten Iteration 31 des Algorithmus durch die Recheneinheit 20 bestimmbar ist. Bei einer Neigung des Beckens ab im Wesentlichen 10° um die Horizontalachse, abhängig von der Nachgiebigkeit der Untergrundstruktur und von etwaigen pathologischen Veränderungen des Steißbeines, übt das Steißbein der Person einen Sitzdruck 39 auf den Flächensensor 11 aus, der im Wesentlichen auf der von den Spitzensitzdrücken 37, 38 gebildeten gemeinsamen Verbindungslinie C liegt. Der Sitzdruck 39 kann hierbei einen Abstand F zu den Spitzensitzdrücken 37, 38 aufweisen. Der Abstandbereich A der Spitzensitzdrücke 37, 38 entspricht in dieser Lage des Beckens dem halben Radius des kreisförmigen Sitz- und Auflagedruck detektierenden Bereichs E der ersten Iteration 31 des Algorithmus.

Der von dem Steißbein auf den Flächensensor 11 ausgeübte Sitzdruck nimmt während des Abrollvorgangs zu, bis zu einem Übergang 40, bei welchem das Kreuzbein der Person einen Auflagedruck auf den Flächensensor 11 ausübt. In horizontaler Liegeposition der Person, d. h. einer Lage des Beckens 90° um die Horizontalachse gekippt, wird der von dem Kreuzbein ausgeübte Auflagedruck im Ursprung 35 maximal. Von der horizontalen Liegeposition der Person ausgehend, übt, bei einer Bewegung dieser zur Seite, d. h. ein seitliches Abrollen deren Beckens in einer gemischten Bewegung oder einer Rotation um die Longitudinalachse, sodass eine Seitenlage eingenommen wird, das Becken Auflagedrücke auf den Flächensensor 11 aus. Diese Auflagedrücke werden in Fig. 4 durch eine Linie 36 veranschaulicht, die eine gemischte Bewegung 30° der Person zur Seite zeigt. Rotiert eine sich in einer horizontalen Liegeposition befindliche Person 180° in einer gemischten Bewegung, würde der Sitz- und Auflagedruck detektierende Bereich lediglich jene Sitz- und Auflagedrücke erfassen, welche innerhalb der ersten 90° dieser Rotation, d. h. innerhalb des kreisförmigen Sitz- und Auflagedruck detektierenden Bereich E der ersten Iteration 31, auftreten. Auf dem Flächensensor 11 auftretende Sitz- und Auflagedrücke, die über diese 90° Rotation und somit über den kreisförmigen Sitz- und Auflagedruck detektierenden Bereich E der ersten Iteration 31 hinausgehen, würden von diesem nicht erfasst werden. Um derartige Bewegungen der Person dennoch zu erfassen, ist die Rechnereinheit 20 dazu ausgebildet, den Sitz- und Auflagedruck detektierenden Bereich E zu speichern. Treten vom Becken ausgeübte Sitz- und Auflagedrücke an einem Randbereich des kreisförmigen Sitz- und Auflagedruck detektierenden Bereichs E der ersten Iteration 31 auf, wird ein zweiter Sitz- und Auflagedruck detektierenden Bereich, welcher in den Figuren nicht dargestellt ist, an den Sitz- und Auflagedruck detektierenden Bereich E angrenzend abgebildet. Der zweite Sitz- und Auflagedruck detektierende Bereich ist hierbei eine Kopie des Sitz- und Auflagedruckdetektierenden Bereichs E. Dieser Vorgang kann durch die Rechnereinheit mehrmalig wiederholt werden, sodass Bewegungen beziehungsweise von dem Becken ausgeübte Sitz- und Auflagedrücke außerhalb des Sitz- und Auflagedruck detektierenden Bereichs E für jede Bewegung der Person erfassbar sind.

Bei weiterem seitlichem Abrollen des Beckens in einer Bewegung um die Longitudinalachse oder einer gemischten Bewegung, d. h. die Person nimmt eine horizontale Bauchlage ein, wird ausschließlich die pelvine Struktur belastet. Das Schambein und die Beckenkämme können maximale Auflagedrücke auf den Flächensensor 11 ausüben. Diese Auflagedrücke entsprechen mathematisch den Auflagedrücken der in Rückenlage befindlichen Person, wobei in der Rückenlage die sakrale Struktur und in der Bauchlage die pelvine Struktur belastet werden. Bei einer maximalen Belastung des Kreuzbeins in horizontaler Rückenlage im Ursprung 35 beziehungsweise des Schambeins in horizontaler Bauchlage in einem Bereich des Übergangs 40, ist die stabile Gleichgewichtslage erreicht.

Die Abstände F und G sind im Wesentlichen gleich groß, wobei diese sich beispielsweise aufgrund einer pathologischen Veränderung der Anatomie der Person im Bereich des Steißbeins, unterscheiden können. Bei ungleichen Abständen F und G, und die Kenntnis über weitere, in den Figuren nicht dargestellte, charakteristischen Punkte beziehungsweise Druckbereiche, kann der Abstand F rechnerisch korrigiert und trotzdem exakte Rückschlüsse auf eine ergonomisch richtige oder falsche Sitz- oder Liegeposition gemacht werden.

Es kann erwähnt werden, dass das erfindungsgemäße Verfahren auch dann mathematisch exakte Ergebnisse liefert, wenn die Sensitivität der Sensoren, beispielsweise aus Kostengründen, auf einen sehr schmalen Bereich reduziert ist und folglich nur kein oder maximale Druckwerte von dem Sensor detektiert werden.

Es kann weiters erwähnt werden, dass der Flächensensor auch nur unmittelbar im Kontaktbereich der Sitzbeinhöcker und des Steiß-/ und Kreuzbeins in die Sitzfläche integriert sein kann. Dies ist möglich, da für das Berechnungsverfahren, ausgehend von einem auswertbaren Parameter, nur davon abhängige geometrische Fixpunkte zur Beurteilung der Beckenlage herangezogen werden, diese aber physisch nicht tatsächlich vorhanden sein müssen. Dies wäre beispielsweise zur Lagebestimmung für reine Sitzsysteme interessant.

## Patentansprüche

1. Verfahren (100) zur Orts- und Lagebestimmung eines Beckens einer auf einem Sitz (10) sitzenden oder auf einer Liege oder einer Auflage sitzenden oder liegenden Person, wobei das Verfahren (100) die folgenden Schritte umfasst:

   a) Erfassen von von den Sitzbeinhöckern und dem Steißbein der im Wesentlichen aufrecht sitzenden Person oder den Beckenkämmen und dem Schambein der in Bauchlage liegenden Person auf den Sitz (10), die Liege oder die Auflage ausgeübten Sitzdrücke/Auflagedrücke durch einen an dem Sitz (10), der Liege oder der Auflage angeordneten Flächensensor (11);
   b) Ermitteln einer ersten Position ($P_1$) auf dem Flächensensor (11) durch eine Rechnereinheit (20), bei der ein von einem ersten Sitzbeinhöcker/Beckenkamm der Person ausgeübter erster Spitzensitzdruck/Spitzenauflagedruck erreicht wird;
   c) Ermitteln einer zweiten Position ($P_2$) auf dem Flächensensor (11) durch die Rechnereinheit (20), bei der ein von einem zweiten Sitzbeinhöcker/Beckenkamm der Person ausgeübter zweiter Spitzensitzdruck/Spitzenauflagedruck erreicht wird, wobei die zweite Position ($P_2$) in einem vorbestimmten Abstandbereich A zu der ersten Position ($P_1$) liegt;
   d) Ermitteln eines Abstandes B zwischen der ersten Position ($P_1$) und der zweiten Position ($P_2$) durch die Rechnereinheit (20);
   e) Ermitteln einer dritten Position ($P_3$) auf dem Flächensensor (11) durch die Rechnereinheit (20), bei der ein von dem Steißbein/Schambein der Person ausgeübter dritter Spitzensitzdruck/Spitzenauflagedruck erreicht wird, wobei die dritte Position ($P_3$) zwischen der ersten Position ($P_1$) und der zweiten Position ($P_2$) liegt, und wobei die dritte Position ($P_3$) im Wesentlichen auf deren gemeinsamen Verbindungslinie (C) liegt oder in einem vorbestimmten Abstandsbereich auf einer im Wesentlichen senkrecht zu der Verbindungslinie (C) verlaufenden Linie (D) liegt;
   f) Einschränken eines Sitz- und Auflagedruck detektierenden Bereichs (E) des Flächensensors (11) durch die Rechnereinheit (20); und
   g) Auswerten der Sitz- und Auflagedrücke des Beckens der Person in dem eingeschränkten Sitz- und Auflagedruck detektierenden Bereich (E) durch die Rechnereinheit (20),

**dadurch gekennzeichnet, dass** die Rechnereinheit (20) zu dem Einschränken des Sitz- und Auflagedruck detektierenden Bereichs (E) einen iterativen Algorithmus ausführt, der die Mandelbrot-Menge (30) abbildet.

2. Verfahren (100) nach Anspruch 1, **dadurch gekennzeichnet, dass** die Iteration beim Erreichen einer vorgegebenen Anzahl unterbrochen wird.

3. Verfahren (100) nach Anspruch 2, **dadurch gekennzeichnet, dass** die Form und die Größe des eingeschränkten Sitz- und Auflagedruck detektierenden Bereichs (E) des Flächensensors (11) von der Anzahl der Iterationen abhängen.

4. Verfahren (100) nach einem der vorangehenden Ansprüche, **dadurch gekennzeichnet, dass** zur Ermittlung der ersten Position ($P_1$), der zweiten Position ($P_2$) und der dritten Position ($P_3$) eine Mustererkennung der Maxima der Sitz- und Auflagedrücke durch die Rechnereinheit (20) durchgeführt wird.

5. Vorrichtung (200) zur Orts- und Lagebestimmung eines Beckens einer auf einem Sitz (10) sitzenden oder auf einer Liege oder einer Auflage sitzenden oder liegenden Person, umfassend eine Rechnereinheit (20) und einen mit der Rechnereinheit (20) verbundenen Flächensensor (11), der auf dem Sitz (10), der Liege oder der Auflage angeordnet und dazu ausgebildet ist, Sitz- und Auflagedrücke, die von dem Becken der Person auf den Flächensensor (11) ausgeübt werden, zu erfassen, wobei die Rechnereinheit (20) dazu ausgebildet ist, die Schritte b) - g) des Verfahrens (100) gemäß Anspruch 1 auszuführen, **dadurch gekennzeichnet, dass** die Rechnereinheit (20) weiters dazu ausgebildet ist, zu dem Einschränken des Sitz- und Auflagedruck detektierenden Bereichs (E) einen iterativen Algorithmus auszuführen, der die Mandelbrot-Menge (30) abbildet.

6. Vorrichtung (200) nach Anspruch 5, **dadurch gekennzeichnet, dass** der Flächensensor (11) ein Array von Sensoren (15) ist, die ausgewählt sind aus der Gruppe bestehend aus mechanischen, elektrischen, pneumatischen oder hydraulischen Sensoren.

7. Vorrichtung (200) nach Anspruch 5 oder 6, **dadurch gekennzeichnet, dass** der Flächensensor (11) so geformt ist, dass dieser sich wenigstens teilweise dem Becken der Person anpasst.

8. Computerprogrammprodukt, umfassend Befehle, die bei der Ausführung des Programms durch die Rechnereinheit (20) der Vorrichtung (200) von Anspruch 5 diese veranlassen, die Schritte b) - g) des Verfahrens (100) gemäß Anspruch 1 auszuführen, **dadurch gekennzeichnet, dass** das Computerprogrammprodukt weiters Befehle umfasst, die bei der Ausführung des Programms durch die Rechnereinheit (20) diese veranlasst, zu dem Einschränken des Sitz- und Auflagedruck detektierenden Bereichs (E) einen iterativen Algorithmus auszuführen, der die Mandelbrot-Menge (30) abbildet.

9. Computerlesbarer Datenträger, auf dem das Computerprogrammprodukt nach Anspruch 8 gespeichert ist.

**Claims**

1. A method (100) of determining the place and location of a pelvis of a person sitting on a seat (10) or sitting or lying on a lounger or a support, wherein the method (100) comprises the following steps:

   a) detecting sitting pressures/contact pressures exerted on the seat (10), the lounger or the support by the ischial tuberosities and the coccyx of the person sitting essentially upright or by the iliac crests and the pubic bone of the person lying in a prone position by means of a surface sensor (11) arranged on the seat (10), the lounger or the support;
   b) determining, by a computer unit (20), a first position ($P_1$) on the surface sensor (11) at which a first peak sitting pressure/peak contact pressure exerted by a first ischial tuberosity/iliac crest of the person is reached;
   c) determining, by the computer unit (20), a second position ($P_2$) on the surface sensor (11) at which a second peak sitting pressure/peak contact pressure exerted by a second ischial tuberosity/iliac crest of the person is reached, the second position ($P_2$) being within a predetermined distance range A from the first position ($P_1$);
   d) determining, by the computer unit (20), a distance B between the first position ($P_1$) and the second position ($P_2$);
   e) determining, by the computer unit (20), a third position ($P_3$) on the surface sensor (11) at which a third peak sitting pressure/peak contact pressure exerted by the coccyx/pubic bone of the person is reached, the third position ($P_3$) being between the first position ($P_1$) and the second position ($P_2$), and the third position ($P_3$) being substantially on their common connecting line (C) or within a predetermined distance range on a line (D) running essentially perpendicularly to the connecting line (C);
   f) limiting, by the computer unit (20), an area (E) of the surface sensor (11) which detects sitting and contact pressures; and
   g) evaluating, by the computer unit (20), the sitting and contact pressures of the person's pelvis in the restricted area (E) detecting sitting and contact pressures,

   **characterized in that,** for narrowing down the area (E) detecting sitting and contact pressures, the computer unit (20) runs an iterative algorithm which depicts the Mandelbrot set (30).

2. A method (100) according to claim 1, **characterized in that** the iteration is interrupted when a predetermined number is reached.

3. A method (100) according to claim 2, **characterized in that** the shape and the magnitude of the restricted area (E) of the surface sensor (11) which detects sitting and contact pressures depend on the number of iterations.

4. A method (100) according to any of the preceding claims, **characterized in that,** for determining the first position ($P_1$), the second position ($P_2$) and the third position ($P_3$), a pattern recognition of the maxima of the sitting and contact pressures is performed by the computer unit (20).

5. A device (200) for determining the place and location of a pelvis of a person sitting on a seat (10) or sitting or lying on a lounger or a support, comprising a computer unit (20) and a surface sensor (11) connected to the computer unit (20) and arranged on the seat (10), the lounger or the support and being designed for detecting sitting and contact pressures which are exerted on the surface sensor (11) by the person's pelvis, with the computer unit (20) being designed for carrying out the steps of the method (100) according to claim 1, wherein the computer unit (20) is configured running an iterative algorithm, which depicts the Mandelbrot set (30), for narrowing down the area (E) detecting the sitting and contact pressure.

6. A device (200) according to claim 5, **characterized in that** the surface sensor (11) is an array of sensors (15) selected from the group consisting of mechanical, electrical, pneumatic or hydraulic sensors.

7. A device (200) according to claim 5 or 6, **characterized in that** the surface sensor (11) is shaped in such a way that it adapts at least partially to the person's pelvis.

8. A computer program product, comprising commands which, when the computer unit (20) of the device (200) of claim 5 executes the program, prompt said computer unit to carry out the steps b) - g) of the method (100) according to claim 1, wherein the computer program unit further comprises commands which, when the computer unit (20) executes the program, prompt it to run an iterative algorithm which depicts the Mandelbrot set (30) for narrowing down the area (E) detecting sitting and contact pressures.

9. A computer-readable data carrier on which the computer program product according to claim 8 is stored.

## Revendications

1. Procédé (100) pour déterminer l'emplacement et la position du bassin d'une personne assise sur un siège (10) ou assise ou allongée sur une couchette ou un support, le procédé (100) comprenant les étapes suivantes :

   a) détecter les pressions d'assise/d'appui exercées sur le siège (10), la couchette ou le support par les ischions et le coccyx de la personne assise sensiblement en position verticale ou par les crêtes iliaques et le pubis de la personne allongée sur le ventre, à l'aide d'un capteur de surface (11) qui est agencé sur le siège (10), la couchette ou le support ;
   b) déterminer à l'aide d'une unité de calcul (20) une première position ($P_1$) sur le capteur de surface (11) dans laquelle un premier pic de pression d'assise/d'appui exercée par un premier ischion/crête iliaque de la personne est atteint ;

   c) déterminer à l'aide de l'unité de calcul (20) une deuxième position ($P_2$) sur le capteur de surface (11) dans laquelle un deuxième pic de pression d'assise/d'appui exercée par un deuxième ischion/crête iliaque de la personne est atteint, dans lequel la deuxième position ($P_2$) se situe à l'intérieur d'une plage de distance A prédéterminée par rapport à la première position ($P_1$) ;
   d) déterminer à l'aide de l'unité de calcul (20) une distance B entre la première position ($P_1$) et la deuxième position ($P_2$) ;
   e) déterminer à l'aide de l'unité de calcul (20) une troisième position ($P_3$) sur le capteur de surface (11) dans laquelle un troisième pic de pression d'assise/d'appui exercée par le coccyx/pubis de la personne est atteint, dans lequel la troisième position ($P_3$) se situe entre la première position (P1) et la deuxième position ($P_2$), et dans lequel la troisième position ($P_3$) se situe sensiblement sur leur ligne de jonction commune (C) ou se situe à l'intérieur d'une plage de distance prédéterminée sur une ligne (D) qui est sensiblement perpendiculaire à la ligne de jonction (C) ;
   f) limiter à l'aide de l'unité de calcul (20) une zone de détection de pression d'assise et d'appui (E) du capteur de surface (11) ; et
   g) évaluer à l'aide de l'unité de calcul (20) des pressions d'assise et d'appui du bassin de la personne dans la zone de détection de pression d'assise et d'appui limitée (E),

   **caractérisé en ce que** l'unité de calcul (20) exécute un algorithme itératif qui reproduit l'ensemble de Mandelbrot (30) pour limiter la zone de détection de pression d'assise et d'appui (E).

2. Procédé (100) selon la revendication 1, **caractérisé en ce que** l'itération est interrompue lorsqu'un nombre prédéterminé est atteint.

3. Procédé (100) selon la revendication 2, **caractérisé en ce que** la forme et la taille de la zone de détection de pression d'assise et d'appui limitée (E) du capteur de surface (11) dépendent du nombre d'itérations.

4. Procédé (100) selon l'une quelconque des revendications précédentes, **caractérisé en ce que**, pour déterminer la première position (P1), la deuxième position (P2) et la troisième position (P3), l'unité de calcul (20) exécute une reconnaissance de forme des maxima des pressions d'assise et d'appui.

5. Dispositif (200) pour déterminer l'emplacement et la position du bassin d'une personne assise sur un siège (10) ou assise ou couchée sur une couchette ou un support, comprenant une unité de calcul (20) et un capteur de surface (11) qui est relié à l'unité de calcul (20), qui est agencé sur le siège (10), la couchette ou le support et qui est conçu de manière à détecter des pressions d'assise et d'appui exercées par le bassin de la personne sur le capteur de surface (11), l'unité de calcul (20) étant conçue de manière à exécuter les étapes b) à g) du procédé (100) selon la revendication 1, **caractérisé en ce que** l'unité de calcul (20) est en outre conçue de manière à exécuter un algorithme itératif qui reproduit l'ensemble de Mandelbrot (30) pour limiter la zone de détection de pression d'assise et d'appui (E).

6. Dispositif (200) selon la revendication 5, **caractérisé en ce que** le capteur de surface (11) est un réseau de capteurs (15) qui sont sélectionné dans le groupe comprenant des capteurs mécaniques, des capteurs électriques, des capteurs pneumatiques ou des capteurs hydrauliques.

7. Dispositif (200) selon la revendication 5 ou 6, **caractérisé en ce que** le capteur de surface (11) présente une forme telle que ce dernier s'adapte au moins partiellement au bassin de la personne.

8. Produit de programme d'ordinateur contenant des instructions qui, lorsque le programme est exécuté par l'unité de calcul (20) du dispositif (200) selon la revendication 5, amènent celle-ci à exécuter les étapes b) à g) du procédé (100) selon la revendication 1, **caractérisé en ce que** le produit de programme d'ordinateur contient en outre des instructions qui, lorsque le programme est exécuté par l'unité de calcul (20), amènent celle-ci à exécuter un algorithme itératif reproduisant l'ensemble de Mandelbrot (30) pour limiter la zone de détection de pression d'assise et d'appui (E).

9. Support de données lisible par ordinateur sur lequel le produit de programme d'ordinateur selon la revendication 8 est enregistré.

100

| 101 | 102 |
|---|---|
| Erfassen von Sitz- und Auflagedrücken durch einen Flächensensor | Ermitteln einer ersten Position auf dem Flächensensor durch eine Rechnereinheit |

| 104 | 103 |
|---|---|
| Ermitteln eines Abstands zwischen der ersten Position und der zweiten Position durch die Rechnereinheit | Ermitteln einer zweiten Position auf dem Flächensensor durch die Rechnereinheit |

| 105 | 106 |
|---|---|
| Ermitteln einer dritten Position auf dem Flächensensor durch die Rechnereinheit | Einschränken eines Sitz- und Auflagedruck detektierenden Bereichs durch die Rechnereinheit |

| | 107 |
|---|---|
| | Auswerten der Sitz- und Auflagedrücke in dem eingegrenzten Bereich durch die Rechnereinheit |

## Fig. 1

200     10

13

12

14

11

15

20

21

## Fig. 2

12

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102012017681 A1
- US 20020193707 A1
- EP 1093755 A1
- JP 2002005761 A

**In der Beschreibung aufgeführte Nicht-Patentliteratur**

- **VAN GEFFEN et al.** *J. Biomech.,* 2009, vol. 42, 1288-1295